Europäisches Patentamt

**European Patent Office**  (11) Veröffentlichungsnummer: **0 006 499**

**Office européen des brevets**  **A1**

(12) **EUROPÄISCHE PATENTANMELDUNG**

(21) Anmeldenummer: **79101812.0**

(22) Anmeldetag: **07.06.79**

(51) Int. Cl.³: **A 61 B 10/00,** G 01 S 7/00, G 08 C 21/00

(30) Priorität: **15.06.78 DE 2826263**

(71) Anmelder: **SIEMENS AKTIENGESELLSCHAFT Berlin und München, Postfach 261, D-8000 München 22 (DE)**

(43) Veröffentlichungstag der Anmeldung: **09.01.80 Patentblatt 80/1**

(72) Erfinder: **Weyns, Arthur, Dipl.-Ing., "Ten Weynranken", Willendries 5, B-2960 Muizen (BE)**
Erfinder: **Walz, Alfred, Tannenweg 14, D-8501 Burgthann (DE)**

(84) Benannte Vertragsstaaten: **AT CH FR GB NL**

(54) **Gerät zur Objektabtastung.**

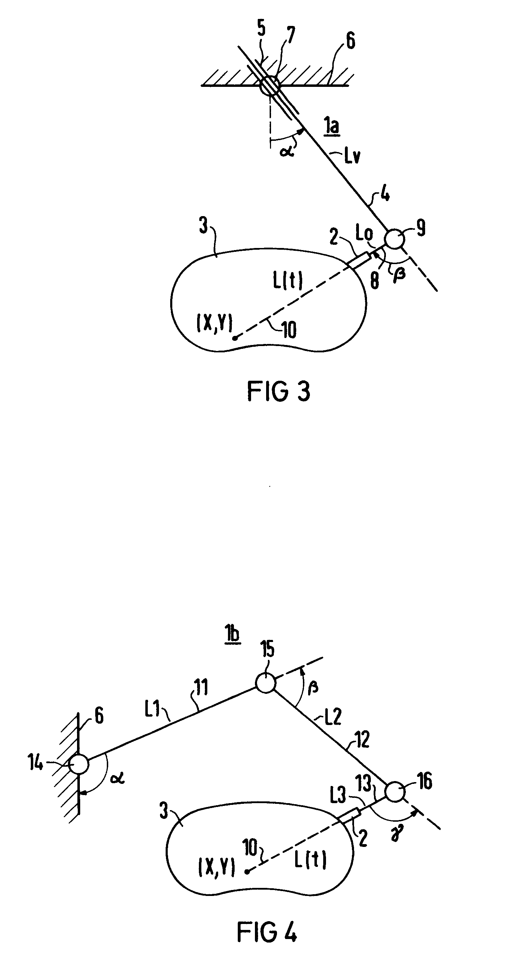

(57) Die Erfindung bezieht sich auf ein Gerät zur Objektabtastung, insbesondere Ultraschall-Abtastgerät, mit Gelenkträger (1) für wenigstens einen Abtastkopf (2), insbesondere Ultraschallkopf, und mit einer Einrichtung mit Winkelgebern in den Gelenken (7, 9) des Gelenkträgers zur Erfassung von Punkt- und/oder Richtungskoordinaten der Abtastung bei beliebiger Raumstellung des Abtastkopfes (2) in Abhängigkeit von Winkelsignalen des Winkelgebers. Erwünscht ist eine Winkelerfassung und -verarbeitung, die gegenüber dem Stand der Technik erheblich vereinfacht und dennoch hochgenau, störunempfindlich und preisgünstig ist. Gemäß der Erfindung ist dies erreicht durch wenigstens je einen Synchronmotor (17, 18) vorzugsweise in jedem winkelzuerfassenden Gelenk (7, 9) des Gelenkträgers (1) als Winkelgeber für den Sinus und/oder Cosinus des Winkelwertes des Drehwinkels (α, β) der Motorachse sowie durch eine rein elektrische Zusammenschaltung einzelner oder mehrerer Synchronmotoren im Sinne der Bildung einer Drehmelder- oder Resolverkette (17, 18) oder einer gemischten Kette aus Drehmeldern und Resolvern zur Umwandlung von Sinus- bzw. Cosinuswerten erfaßter Drehwinkel in Summen und/oder Differenzen aus Einzelwinkeln und/oder in Sinus- bzw. Cosinusfunktionen solcher Winkelsummen oder Winkeldifferenzen (Fig. 3).

SIEMENS AKTIENGESELLSCHAFT      Unser Zeichen
Berlin und München              VPA 78 P 5057 EUR

Gerät zur Objektabtastung

Die Erfindung bezieht sich auf ein Gerät zur Objektabtastung, insbesondere Ultraschall-Abtastgerät, mit Gelenkträger für wenigstens einen Abtastkopf, insbesondere Ultraschallkopf, und einer Einrichtung mit Winkelgebern in den Gelenken des Gelenkträgers zur Erfassung von Punkt- und/oder Richtungskoordinaten der Abtastung bei beliebiger Raumstellung des Abtastkopfes in Abhängigkeit von Winkelsignalen der Winkelgeber.

Unter derartigen Geräten sind alle solche zu verstehen, die in irgendeiner Form Abtastungen von Untersuchungsobjekten im Sinne beispielsweise der Bestimmung des Objektumrisses, der Erfassung von Objektschnittebenen, der Ermittlung von Richtung bzw. Distanz zu anzupeilenden Objekten od.dgl. erlauben. Alle diese Abtastformen laufen hinaus auf die Erfassung von Punkt- und/oder Richtungskoordinaten der jeweiligen Abtastung. Die Abtastung kann direkt durch mechanischen Abtastkopf am

Kue 5 Kli / 23.5.1978

0006499

Objekt erfolgen. Sie kann aber auch durch Abtastmedien, wie Licht (z.B. Laser), Wärmestrahlung, Schall
od.dgl., erfolgen. Von besonderem Interesse ist die
Abtastung mittels Ultraschall, wobei dann also das
Gerät zur Objektabtastung ein Ultraschall-Abtastgerät
und der Abtastkopf ein Ultraschallkopf sind.

Insbesondere hinsichtlich Ultraschallabtastung sind
nun Ultraschall-Schnittbildgeräte bekannt, die zur Erfassung von Punkt- und/oder Richtungskoordinaten der
Ultraschallabtastung entweder die Winkelmessung bezogen auf eine Horizontale oder Vertikale oder die Winkelmessung zwischen den Gelenkstangen des Gelenkträgers
(eingeschlossener Winkel bzw. Supplement dieses Winkels) benutzen.

Die Winkelmessung bezogen auf eine Horizontale oder
Vertikale (z.B. gemäß DE-AS 15 73 745 oder auch deutsche Patentanmeldung P 27 11 098.1) erlaubt zwar wegen
der einfachen Koordinatenbeziehungen den Einsatz von
Winkelgebern direkt für den Winkel oder solchen für den
Sinus bzw. Cosinus des Winkels (Sinus-Cosinus-Potentiometer als Winkelgeber); die Änderung der Winkel muß jedoch mechanisch zu einem festen Referenzpunkt, z.B. mittels Seil oder mittels eines Pantographsystems ähnlich
dem einer Zeichenmaschine, übertragen werden. Das mechanische System muß hochgenau und gegen Temperatureinflüsse oder auch Schlupf störunempfindlich sein. Diese Bedingungen verteuern selbstverständlich das Gesamtsystem.

Die Winkelmessung zwischen den Gelenkstangen gelingt
analog bisher nur mit einer Serienschaltung von Potentiometern oder digital mit inkrementalen Winkelgebern. Eine Serienschaltung aus Potentiometern benötigt jedoch viel Raum; sie ist außerdem teuer und sehr temperaturempfindlich. Digitale Winkelmesser sind ebenfalls kostenaufwendig sowie temperatur- und störempfindlich. Sie setzen darüber hinaus die Anwendung eines Rechners voraus (z.B. gemäß dem Artikel "A Computer-Controlled Ultrasound Image-Forming System" von S.Schorum, H.Fidel aus Searle Ultrasound, Santa Clara, California).

Aufgabe vorliegender Erfindung ist es, bei einem Gerät zur Objektabtastung der eingangs genannten Art eine Einrichtung zur Erfassung von Punkt- und/oder Richtungskoordinaten der Abtastung vorzusehen, die mit gegenüber dem Stand der Technik erheblich vereinfachter und dennoch hochgenauer, störunempfindlicher und preisgünstiger Winkelmessung auskommt.

Die Aufgabe wird erfindungsgemäß gelöst durch wenigstens je einen Synchronmotor vorzugsweise in jedem winkelzuerfassenden Gelenk des Gelenkträgers als Winkelgeber für den Sinus und/oder Cosinus des Winkelwertes des Drehwinkels der Motorachse sowie durch eine rein elektrische Zusammenschaltung einzelner oder mehrerer Synchronmotoren im Sinne der Bildung einer Drehmelder- oder Resolverkette oder einer gemischten Kette aus Drehmeldern und Resolvern zur Umwandlung von Sinus- bzw. Cosinuswerten erfaßter Drehwinkel in Summen und/oder Differenzen aus Einzelwinkeln und/oder in Sinus- bzw. Cosinusfunktionen solcher Winkelsummen bzw. Winkeldifferenzen.

Die Erfindung arbeitet mit Synchronmotoren als Winkelgeber, die durch einfache Serienschaltung, also auf rein elektrische Weise, als Drehmelder- oder Resolverkette die erwünschten Winkelkombinationswerte liefern.

Synchronmotoren sind ferner sehr stabil, störunanfällig, sie weisen kleine räumliche Abmessungen auf und sind im Verhältnis zu digitalen Winkelgebern preisgünstiger. Im Gegensatz zu inkrementalen digitalen Systemen liegt mit dem Einschalten sofort die richtige Winkelinformation vor. Der Einsatz von Synchronmotoren im erfindungsgemäßen Sinne vereinfacht also nicht nur die Winkelgabe in erheblicher Weise; die gesamte Winkelmessung ist sehr genau, sie ist störunempfindlicher und der Einsatz der neuen Winkelmesser ist insgesamt preisgünstiger.

Schaltungen von Synchronmotoren in Serie als Drehmelder- oder auch Resolverkette sind an sich nicht mehr neu. Derartige Ketten sind beispielsweise aus "Technical Information For The Engineer" der Singer Company, Little Falls, USA, 1967 bzw. aus der Siemens-Schrift "Siemens-Drehmelder" aus dem Jahre 1970 vorbekannt. Festzuhalten ist jedoch, daß sämtliche der beschriebenen Anwendungsmöglichkeiten für Drehmelder- bzw. Resolverketten sich auf sog. Winkel-Nachlaufsteuerungen beschränken. Solche Nachlaufsteuerungen arbeiten im Prinzip so, daß ein bestimmtes Drehobjekt, das sich in gewisser Entfernung befindet, auf einen bestimmten Winkel nachgestellt werden soll (Anwendung z.B. Radartechnik, Richtfunktechnik od.dgl.). In einem solchen Falle dienen die Motoren der Drehwinkel- oder auch Resolverkette zur Übertragung dieses Winkels (oder auch Winkelsummen bzw. Differenzen), der zu diesem Zwecke in den jeweils ersten Motor der Kette eingespeist wird und von dort naturgetreu dem jeweils letzten Motor der Kette als Drehantrieb für das zu drehende Objekt übermittelt wird. Die Drehmelder- bzw. Resolverketten des Standes der Technik unterscheiden sich also von vorliegender Erfindung grundsätzlich nach Aufgabenstellung und Lösung.

Weitere Vorteile und Einzelheiten der Erfindung ergeben sich aus der nachfolgenden Beschreibung von Ausführungsbeispielen anhand der Zeichnung in Verbindung
mit den Unteransprüchen.

Es zeigen:

Fig. 1 und 2 Prinzipschaltungen einer Resolverkette zur Ermittlung des Sinus bzw. Cosinus
von Winkelsummen einerseits und Winkeldifferenzen andererseits,

Fig. 3 das Prinzipschaltbild einer Objektabtastung
(im vorliegenden Fall Ultraschall-Compound-
Scan) mit zweiarmigem Gelenkträger für einen
Abtastkopf,

Fig. 4 das Prinzipschaltbild einer entsprechenden
Abtastung mittels dreiarmigem Gelenkträger,

Fig. 5 ein auf die Abtastung mittels zweiarmigem
Gelenkträger gemäß Fig. 3 zugeschnittenes
Ausführungsbeispiel mit Resolverkette für ein
Ultraschall-Abtastgerät im Prinzipschaltbild,

Fig. 6 ein bevorzugtes Ausführungsbeispiel für die
Demodulatoren beispielsweise des Prinzipschaltbildes der Fig. 5,

Fig. 7 ein Diagramm der Spannungsverläufe zur Steuerung der Demodulatoren der Fig. 6,

Fig. 8 ein Ausführungsbeispiel für eine Ultraschall-
Abtastung mit einem dreiarmigem Gelenkarm
gemäß Fig. 3, der ebenfalls eine Resolverkette beinhaltet,

Fig. 9 und 10 Prinzipschaltbilder für Drehmelderketten,

Fig.11 das Prinzipschaltbild eines Ultraschall-
Abtastgerätes für Ultraschallabtastung mit
zweiarmigem Gelenkträger gemäß Fig. 3, der
eine Drehmelderkette umfaßt,

Fig.12 das Prinzipschaltbild eines Abtastgerätes
für Ultraschallabtastung mittels dreiarmigem Gelenkträger gemäß Fig. 4, der ebenfalls eine Drehmelderkette beinhaltet.

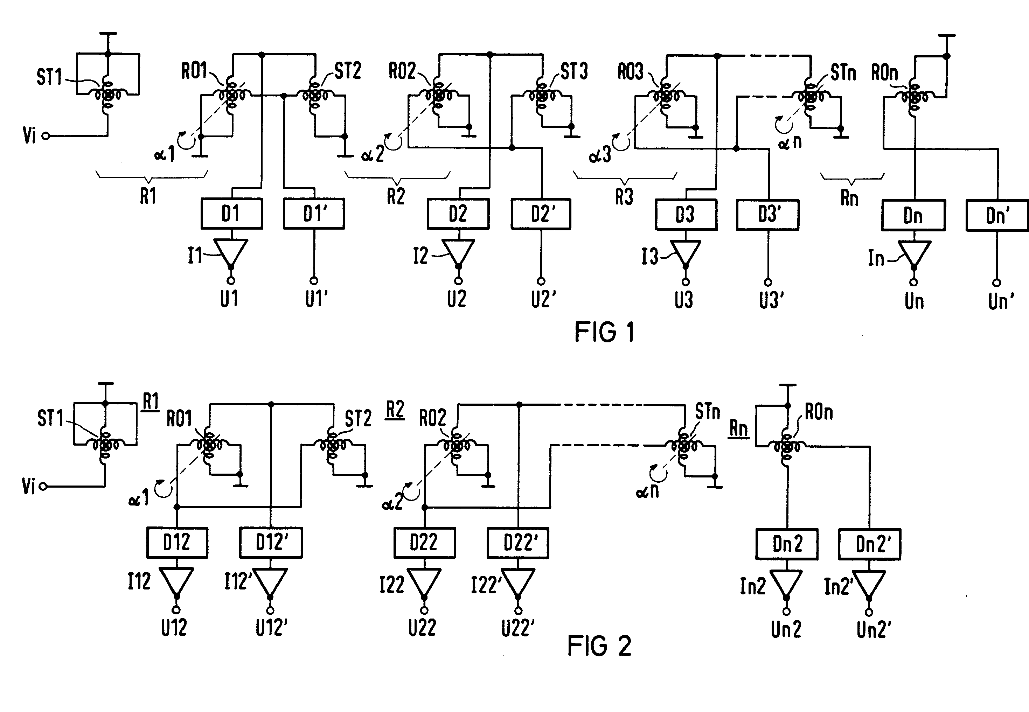

In den Resolverketten der Fig. 1 und 2 sind die Statorwicklungen mit den zugehörigen Rotorwicklungen eines jeden Synchronmotors in der Serienschaltung von links nach
rechts mit steigender Nummer jeweils mit ST1, RO1; ST2,
RO2; ST3, RO3 ... STn, ROn bezeichnet. Die einzelnen
Resolver tragen die Bezeichnungen R1, R2, R3 ... Rn.
Die Speisewechselspannung der Kette ist mit Vi angegeben. Die Drehwinkel der einzelnen Rotoren sind mit
$\alpha$ 1, $\alpha$ 2, $\alpha$ 3 ... $\alpha$ n bezeichnet.

In der speziellen Zusammenschaltung der Fig. 1 ergeben
sich nach Demodulation in Demodulatoren D1, D1'; D2,
D2'; D3, D3' ... Dn, Dn' und Invertierung in Invertiergliedern I1, I2, I3 ... In die Cosinus- bzw. Sinusfunktionen von Winkeln und Winkelsummen wie folgt:

$$U1 = k1 \cos \alpha 1$$
$$U1' = k1 \sin \alpha 1$$

$$U2 = k1 \, k2 \cos ( \alpha 1 + \alpha 2 )$$
$$U2' = k1 \, k2 \sin ( \alpha 1 + \alpha 2 )$$

$$U3 = k1\ k2\ k3\ \cos(\alpha 1 + \alpha 2 + \alpha 3)$$
$$U3' = k1\ k2\ k3\ \sin(\alpha 1 + \alpha 2 + \alpha 3)$$

$$Un = k1\ k2\ ...\ kn\ \cos(\alpha 1 + \alpha 2 + ... + \alpha n)$$
$$Un' = k1\ k2\ ...\ kn\ \sin(\alpha 1 + \alpha 2 + ... + \alpha n)$$

Entsprechend ergeben sich im Zuschnitt der Fig. 2 nach Demodulation in den Demodulatoren D12, D12'; D22, D22' ... Dn2, Dn2' und Invertierung in den Invertiergliedern I12, I12'; I22, I22' ... In2, In2' die Sinus- bzw. Cosinusfunktionen von Winkeln oder Winkeldifferenzen wie folgt:

$$U12 = k1\ \sin\alpha 1$$
$$U12' = k1\ \cos\alpha 1$$

$$U22 = k1\ k2\ \sin(\alpha 1 - \alpha 2)$$
$$U22' = k1\ k2\ \cos(\alpha 1 - \alpha 2)$$

$$Un2 = k1\ k2\ ...\ kn\ \sin(\alpha 1 - \alpha 2 - ... - \alpha n)$$
$$Un2' = k1\ k2\ ...\ kn\ \cos(\alpha 1 - \alpha 2 - ... - \alpha n)$$

k1, k2, k3 ... kn sind in beiden Fällen die Übertragungsfaktoren der einzelnen Resolver R1, R2 ... Rn.

Die Fig. 3 zeigt nun das Prinzipschaltbild einer Objektabtastung (im vorliegenden Fall Ultraschall-Compound-Scan) mit zweiarmigem Gelenkträger 1a für einen Abtastkopf 2 (Ultraschall-Abtastkopf). Speziell beim Compound-Scan müssen während der Abtastung laufend die Positionen des Schallkopfes 2 auf der Oberfläche des Untersuchungsobjektes 3 (menschlicher Körper) sowie die Einstrahlrichtung in das Objekt erfaßt werden, da die Echos der Schallimpulse längs jedes Schallstrahles ortsgetreu als helligkeitsmodulierte Linien auf dem Bildschirm einer Oszillographenröhre wiedergegeben werden sollen.

Speziell im Ausführungsbeispiel der Fig. 3 erfolgt die Koordinatenerfassung in der Abtastebene mittels einer Längen- und zwei Winkelmessungen. Der Doppel-Gelenkarm 1a besteht demnach aus einem ersten Armteil 4, das in einer Längsführung 5 an einer Armhalterung 6 (Boden- oder Deckenstativ) längsverschiebbar und mittels erstem Drehgelenk 7 um den Winkel $\alpha$ drehbar angeordnet ist. Die Länge des ersten Armteiles ist mit Lv bezeichnet. Als Träger für den Ultraschallkopf 2 dient ein zweites Armteil 8, das am ersten Armteil 1 mittels zweitem Drehgelenk 9 drehbar angelenkt ist. Die Länge des zweiten Armteiles ist mit Lo, der Drehwinkel entsprechend mit $\beta$ bezeichnet. Im Untersuchungsobjekt 3 laufen ein ausgesendeter Ultraschallimpuls bzw. die aufgrund dieses Schallimpulses rücklaufenden Echoimpulse entlang der Strahlrichtung 10. Ein beliebiger Punkt auf dieser Strahlrichtung 10 hat die Koordinaten X, Y. Die Tiefe des jeweiligen Punktes X, Y im Objekt 3 ist mit L (t) bezeichnet. Da bei der Echoabbildung am Bildschirm der Oszillographenröhre sämtliche Echokoordinaten entlang der Strahlrichtung 10 abgebildet werden sollen, ist L (t) demnach eine lineare Funktion der Zeit (Sägezahnfunktion).

Mit dem dargestellten Doppelgelenkarm als Träger für den Ultraschall-Abtastkopf 2 ergeben sich in Abhängigkeit von den Längen Lv und Lo der Armteile sowie den jeweils eingestellten Winkeln $\alpha$ und $\beta$ die Abtastkoordinaten für einen beliebigen Abtastpunkt im Objekt 3 wie folgt:

$$X = Lv \sin \alpha + [Lo + L(t)] \sin (\alpha + \beta)$$
$$Y = Lv \cos \alpha + [Lo + L(t)] \cos (\alpha + \beta)$$

Entsprechend ergeben sich für den dreiarmigen Gelenkträger 1b der Fig. 4 die laufenden Koordinatenbeziehungen zu:

$$X = L1 \sin \alpha + L2 \sin (\alpha + \beta) + [L3 + L(t)] \sin (\alpha + \beta + \gamma)$$

$$Y = L1 \cos \alpha + L2 \cos (\alpha + \beta) + [L3 + L(t)] \cos (\alpha + \beta + \gamma),$$

wobei L1, L2, L3 wieder die Längen der einzelnen Arme 11, 12 und 13 des Gelenkträgers, L(t) die Tiefenposition des Koordinatenpunktes X, Y im Untersuchungsobjekt 3 und die Winkel $\alpha$, $\beta$ und $\gamma$ Drehwinkel für die Drehung der einzelnen Arme um Drehgelenke 14, 15 und 16 sind.

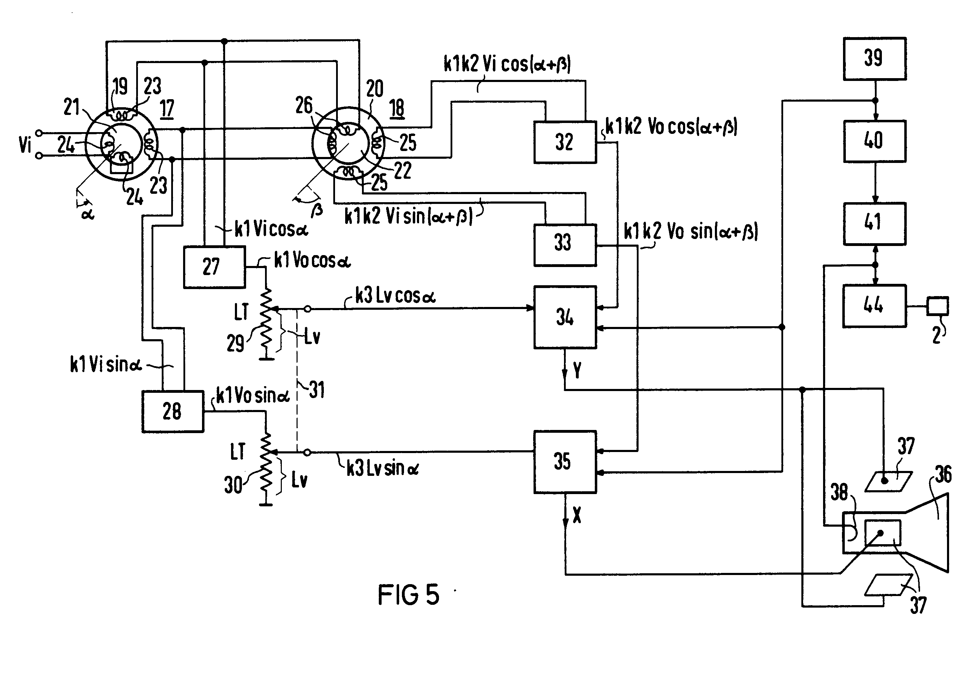

Die Fig. 5 zeigt nun ein auf den Doppelgelenkarm der Fig. 3 zugeschnittenes Ausführungsbeispiel mit Resolverkette für ein Ultraschall-Abtastgerät. Gemäß den beiden Drehgelenken 7 und 9 des Doppelgelenkarmes der Fig. 3 umfaßt der Arm insgesamt zwei Synchronmotoren 17 und 18, bestehend aus den Statoren 19 bzw. 20 und den Rotoren 21 bzw. 22. Der Motor 17 beinhaltet für seinen Stator entsprechend die Statorwicklungen 23 und für seinen Rotor 21 die Rotorwicklungen 24. Entsprechend umfaßt der Motor 18 für den Stator 20 die Statorwicklungen 25 und für den Rotor 22 die Rotorwicklungen 26. Die beiden Synchronmotoren 17 und 18 sind als Winkelgeber in den Drehgelenken 7 und 9 des Doppelgelenkarmes der Fig. 3 eingesetzt. Die Motoren fungieren als Resolver und bilden also in der dargestellten Zusammenschaltung eine Resolverkette.

Entsprechend der Darstellung der Fig. 5 werden die Cosinus- und Sinusfunktion des Winkels $\alpha$ im Gelenk 7 mittels des Resolvers 17 erfaßt. Die beiden Ausgänge dieses Resolvers liefern die Ausgangsspannungen k1 Vi cos $\alpha$ bzw. k1 Vi sin $\alpha$ , wobei, wie oben schon erläutert, k1 wieder der Übertragungsfaktor des Resolvers 17 und Vi das Eingangssignal sind. Da Synchronmotoren ausschließlich mit Wechselspannung betrieben werden, ist das Eingangssignal Vi ein Wechselsignal nach der Beziehung Vi = Vo · sin $\omega$ t. Bei den Ausgangssignalen des Resolvers 17 ist die Winkelinformation in der Amplitude des Wechselsignals enthalten. Durch Demodulatoren 27 und 28 erhält man (entsprechend Prinzipschaltbild der Fig. 1) schließlich Gleichspannungssignale k1 Vo sin $\alpha$ und k1 Vo cos $\alpha$ . Diese Signale werden in je ein Potentiometer 29 bzw. 30 eingespeist, das ein der Länge Lv des ersten längsverschiebbaren Gelenkarmes entsprechendes Ausgangssignal erzeugt. Die mechanische Kopplung beider Potentiometer 29 und 30 mit der Längenverschiebung des Armes 4 ist mit 31 angedeutet. Mit dem Einstellen der Potentiometer auf die Länge Lv erhält man die gewünschten Faktoren Lv cos $\alpha$ und Lv sin $\alpha$ multipliziert mit einer Konstanten k3. k3 ist hierbei das Verhältnis von k1 Vo zur Gesamtlänge LT des Armes 4 (Gesamtwiderstand der Potentiometer 29 bzw. 30).

Werden nun außerdem die Ausgangssignale des Resolvers 17 in der dargestellten Weise auch noch dem Resolver 18, der sich im Drehgelenk 9 befindet, als Eingangssignale zugeleitet, so erhält man aus der Serienschaltung beider Resolver 17 und 18 zwei Ausgangssignale, deren Amplitude die Cosinus- und Sinusfunktion der Winkelsumme enthält. Die Konstante k2 ist dabei wieder der Übertragungsfaktor des Resolvers 18. Nach Demodulation in den Demodulatoren 32 und 33 stehen die Größen k1 k2 sin ( $\alpha + \beta$ ) und k1 k2 cos ( $\alpha + \beta$ ) zur Verfügung. Werden, wie dargestellt, diese Signale nun in je einen Integrator 34 und 35 als Eingangssignal und die beiden anderen Signale k3 Lv sin $\alpha$ und k3 Lv cos $\alpha$ in die Integratoren als Anfangsbedingungen eingespeist, so stellen die Ausgangssignale der Integratoren 34 und 35 bei entsprechender Wahl der Verstärkung und der Konstanten der Signale für Anfangsbedingung und Integration die beiden Koordinatenformeln für X und Y dar. Werden diese X- und Y-Signale an die Ablenkplatten (oder Ablenkspulen) einer Kathodenstrahlröhre angelegt, so erhält man bei entsprechender Helligkeitsmodulation in Abhängigkeit von anfallenden Echosignalen das erwünschte koordinatengerechte Ultraschallbild. In der Fig. 5 ist die Kathodenstrahlröhre mit 36 bezeichnet; die Ablenkplatten (oder Ablenkspulen) der Röhre sind mit 37 und die Helltastkathode mit 38 angedeutet. Die Beschaltungsblöcke 39, 40, 41 und 44 bezeichnen die üblichen Grundelemente eines Ultraschall-Bildgerätes, wie Zeitgeber, Verzögerungsglieder, Impulsgeber, Ultraschall-Sender-Empfänger.

Wie bereits beschrieben, ist beim Ausgangssignal der Resolver 17 bzw. 18 die Winkelinformation in der Amplitude des jeweiligen Wechselsignals enthalten. Die Winkelinformation sollte mit großer Genauigkeit ermittelt werden. Aus diesem Grunde ist auch eine Amplitudendemodulation hoher Güte erwünscht. Ein einfacher Spitzenwertdetektor ist bei diesen Anforderungen nur wenig geeignet, da bei ihm die Vorzeicheninformation der unterschiedlichen Winkelfunktionen verlorengeht und nur durch Zusatzglieder elektronisch regenerierbar ist. Bei kleinen Sinus- und Cosinuswerten ist darüber hinaus die erforderliche Genauigkeit nur mit erheblichem technischen Aufwand erreichbar.

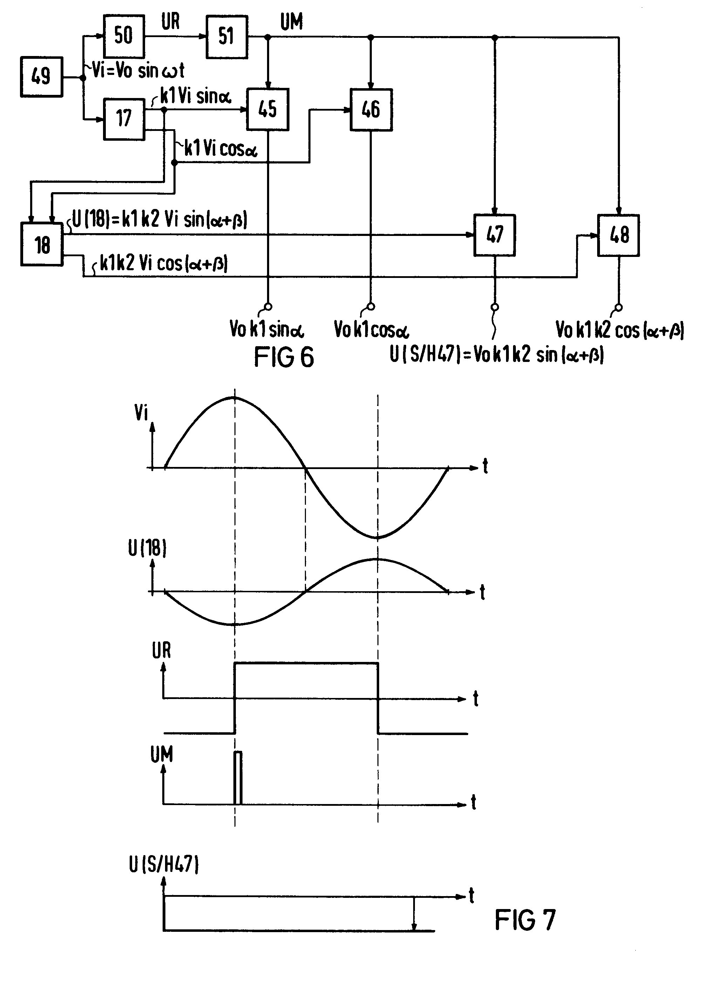

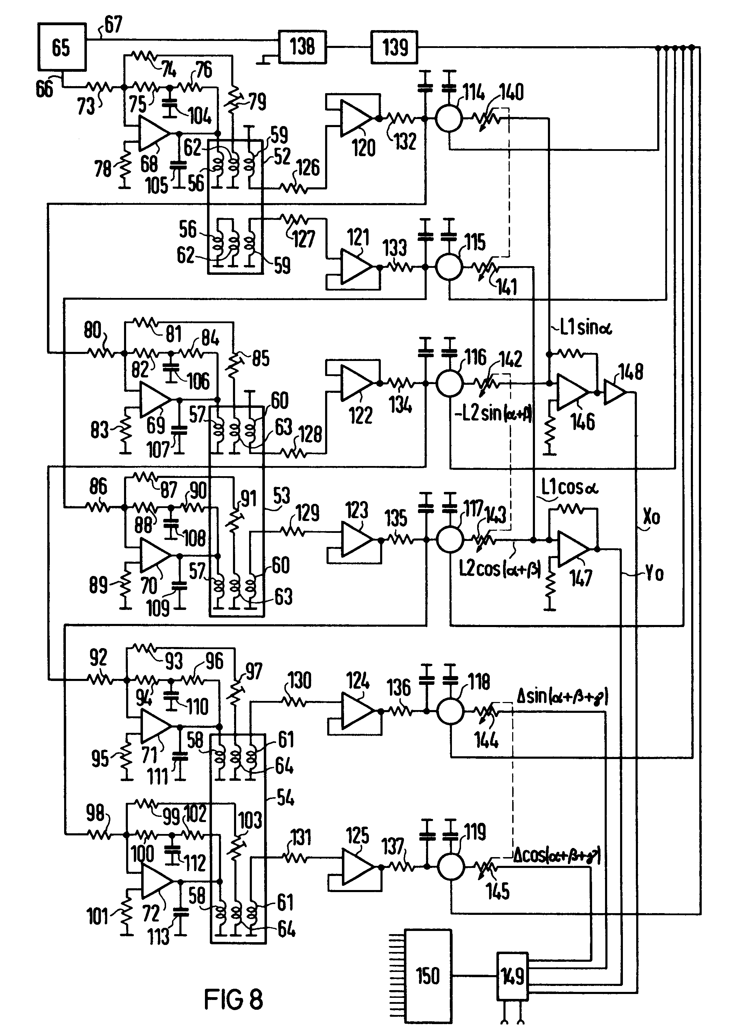

Die Fig. 6 zeigt nun ein Ausführungsbeispiel für eine Demodulatorschaltung zur hochgenauen Amplitudendemodulation. Kernstück ist eine Sample-and-Hold-Schaltung 45 bis 48, deren Einzeltore von der Sinusspannung $Vi = Vo \cdot \sin \omega t$ eines Sinusgenerators 50 über eine PLL (Phased Locked Loop)-Schaltung 51 mit nachgeschalteter monostabiler Kippstufe 52 angesteuert werden. Um Phasenverschiebungen der Resolver-Ausgangssignale gegenüber dieser Sinus-Referenzspannung Vi zu vermeiden, sollten bevorzugt wicklungskompensierte Resolver eingesetzt werden (siehe auch Fig. 8).

Im Ausführungsbeispiel der Fig. 6 wird also eine einzige Referenzspannung Vi der PLL-Schaltung 51 zugeleitet. Die Resonanzfrequenz dieser Schaltung ist gleich der Frequenz des angelegten Referenzsignales Vi. Am Ausgang der PLL-Schaltung 51 fällt somit ein Rechtecksignal UR an, das gegenüber dem Referenzsignal Vi um $90^o$ phasenverschoben ist (Fig. 7). Die positive Flanke dieses Rechtecksignals UR stößt die monostabile Kippstufe 52 an, die daraufhin einen sehr kurzen Rechteckimpuls UM liefert. Dieser Puls UM steuert dann schließlich die Tore 45 bis 48 der Sample-and-Hold-Schaltungen im Sinne der Demodulation der angelegten Signale k1 Vi sin $\alpha$ , k1 Vi cos $\alpha$ , k1 k2 Vi sin ( $\alpha + \beta$ ) und k1 k2 Vi cos ( $\alpha + \beta$ ). Aufgrund dieser Demodulation ergeben sich die erwünschten Sinusfunktionen an den Ausgängen der einzelnen Sample-and-Hold-Tore 45 bis 48. Im Spannungsdiagramm der Fig. 7 ist der Übersicht halber nur eine dieser Gleichspannungen aufgeführt. Es handelt sich um die Ausgangsspannung U(S/H 47) = k1 k2 Vo sin ( $\alpha + \beta$ ) am Ausgang des Tores 47. Die zugehörige Eingangssignalspannung ist die Ausgangsspannung U (18) = k1 k2 Vi sin ( $\alpha + \beta$ ) des Resolvers 18.

Eine PLL-Demodulatorschaltung hat den Vorteil, daß auch große Schwankungen in der Amplitude des Referenzsignals sowie im Fangbereich der PLL-Schaltung liegende Frequenzänderungen die Genauigkeit des Systems kaum oder überhaupt nicht beeinträchtigen. Eine 90°-Phasenverschiebung zwischen Referenz- und Abtastsignal führt dazu, daß die Winkelinformation immer im Maximum des Ausgangssignals gewonnen wird. Da in diesem Augenblick das Ausgangssignal geringste Steilheit aufweist, wird auch die Störempfindlichkeit in diesem Punkt minimal sein. Die PLL-Schaltung ermöglicht darüber hinaus eine polaritätsrichtige Amplitudendemodulation sowie die exakte Auswertung sowohl von großen als auch kleinen Sinus- und Cosinuswerten.

Die Fig. 8 zeigt ein Ausführungsbeispiel für eine Ultraschallabtastung mit einem dreiarmigen Gelenkarm gemäß Fig. 3. Das Prinzipschaltbild der Fig. 8 enthält entsprechend den drei Gelenken 14, 15 und 16 des Gelenkarmes der Fig. 4 jetzt eine Resolverkette aus insgesamt drei Resolvern 52, 53 und 54. Die einzelnen Resolver sind wicklungskompensiert, d.h. sie beinhalten zusätzlich zu den Statorhauptwicklungen 56, 57 bzw. 58 sowie Rotorwicklungen 59, 60, 61 auch noch Kompensationswicklungen 62, 63 bzw. 64 zur Aufhebung von Phasenverschiebungen und Temperatureinflüssen.

Grundspannungserzeuger ist wiederum ein Wechselspannungsgenerator 65, der jedoch im vorliegenden Fall in Modifikation als Sinus-Cosinus-Generator ausgebildet ist. Dementsprechend liefert er also an einem ersten Ausgang 66 eine Sinuswechselspannung und an einem Ausgang 67 eine Cosinusspannung derselben Amplitude und Frequenz wie die Sinusspannung. Die Zuführung der Sinusspannung zum Resolver 52 bzw. der aufgrund Ketten-

schaltung zu den Statorwicklungen der nächstfolgenden Resolver 53 bzw. 54 weitergeleiteten Signale erfolgt über Rückkoppelverstärker 68, 69, 70, 71 sowie 72. Die Rückkoppelverstärker sind in der dargestellten Weise mit ohm'schen Widerständen 73 bis 103 sowie Kapazitäten 104 bis 113 beschaltet. Die Verkopplung der Resolver über die Rotorwicklungen untereinander sowie mit Sample-and-Hold-Demodulatoren 114 bis 119 erfolgt über Impedanzwandler 120 bis 125. Die Impedanzwandler sind in der dargestellten Weise mit ohm'schen Eingangswiderständen 126 bis 131 sowie HF-Entstörgliedern 132 bis 137 beschaltet. Die Ansteuerung der einzelnen Sample-and-Hold-Demodulatorschaltungen 114 bis 119 erfolgt vom Ausgang der Serienschaltung aus einem Komparator für die Rechteckumwandlung der Cosinusschwingung des Generators 65 (anstelle einer PLL-Schaltung) und der monostabilen Kippstufe 139. Über Potentiometer 140 bis 145, die jeweils mechanisch paarweise verkoppelt sind, fallen die im Schaltbild der Fig. 8 angegebenen Signale an. Die Ausgangssignale der Potentiometer 144 und 145 werden dabei direkt, jene der Potentiometer 140 bis 143 über Summenbildner 146 und 147 (wobei dem Summenbildner 146 ein Invertierglied 148 nachgeschaltet ist) einer Multiplexerschaltung 149 zugeleitet. Von dieser Multiplexerschaltung 149 werden dann die Koordinatensignale nach Analog-Digital-Umsetzung in einem A-D-Wandler 150 in Richtung zur Aufzeichnungsröhre, insbesondere über einen Bildspeicher, weitergeleitet.

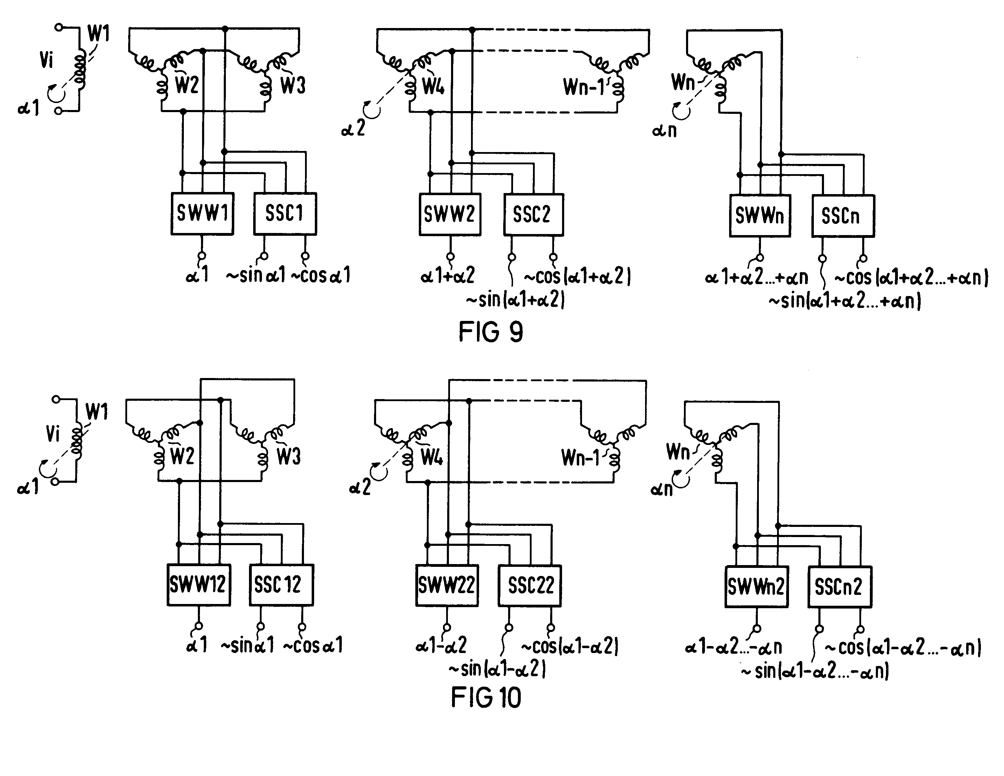

Die Fig. 9 und 10 zeigen Prinzipschaltbilder für Drehmelderketten. Die in den dargestellten Weisen zusammengeschalteten Wicklungen sind mit W1 bis Wn bezeichnet. Vi ist wieder die Eingangswechselspannung und $\alpha_1$, $\alpha_2$ bis $\alpha_n$ sind die Drehwinkel. Nach Zuleitung zu Synchron-Winkel-Wandlern SWW1 bis SWWn ergeben sich nach Schaltungsart der Fig. 9 direkt (analog oder digital) die dargestellten Winkel oder Winkelsummen. Im Ausführungsbeispiel der Fig. 10 fallen entsprechend am Ausgang der Synchron-Winkel-Wandler SWW12 bis SWWn2 Winkel bzw. Winkeldifferenzen an. Anstelle der Direktwinkeldarstellung oder ergänzend zu dieser kann auch Sinus- bzw. Cosinuswinkeldarstellung vorgenommen werden. Zu diesem Zweck dienen die in den Prinzipschaltbildern der Fig. 9 und 10 zusätzlich eingeschalteten Synchron-Sinus-Cosinus-Wandler SSC1 bis SSCn bzw. SSC12 bis SSCn2.

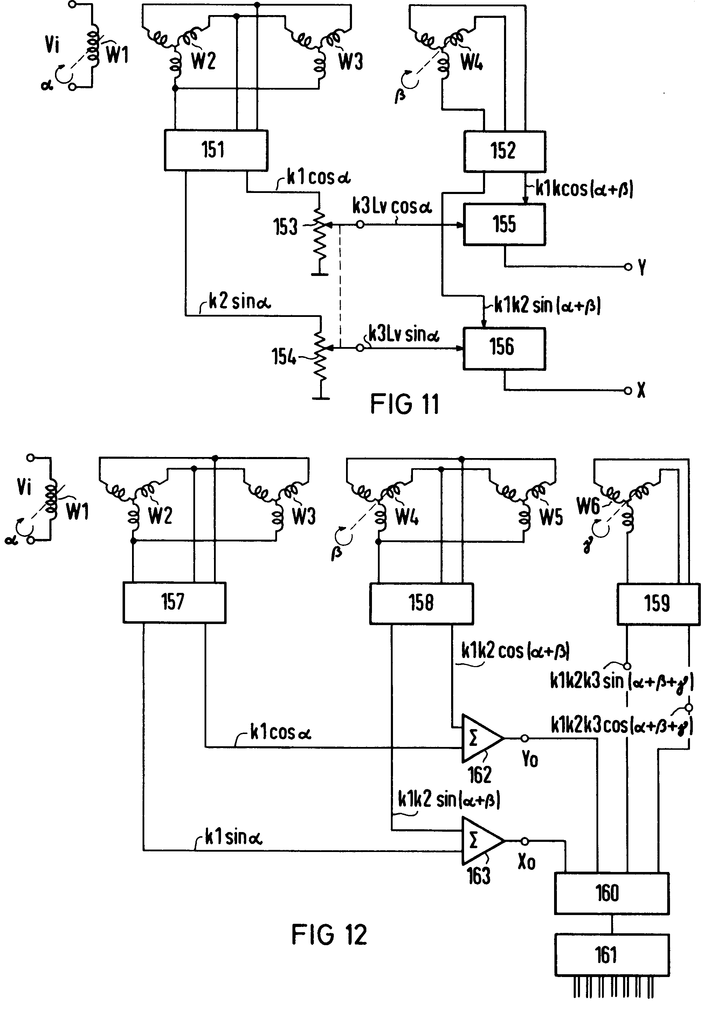

Drehmelderketten mit Synchron-Sinus-Cosinus-Wandlern eignen sich wieder besonders gut für die Winkelgebung bei Abtastung mit zweiarmigem oder dreiarmigem Gelenkträger. Ein Ausführungsbeispiel im Prinzipschaltbild mit Drehmelderkette zur Anwendung bei einem zweiarmigem Gelenkträger gemäß Fig. 3 zeigt Fig. 11. Das Ausführungsbeispiel ähnelt jenem der Fig. 5 mit Resolverkette. Neben der Drehmelderkette W1 bis W4 umfaßt das Ausführungsbeispiel der Fig. 11 also zwei Synchron-Sinus-Cosinus-Wandler 151 und 152. Der Wandler 151 liefert die dargestellten Winkelinformationen, die wieder über Potentiometer 153 und 154 als Anfangsbedingungen Integratoren 155 und 156 zugeführt werden. Die Zuleitung der Winkelsummeninformation erfolgt entsprechend durch Verbindung der Integratoren 155 und 156 mit den Ausgängen des Wandlers 152. Am Ausgang der Integratoren fallen wie gehabt die Informationen für die X-Koordinate und die Y-Koordinate an.

Die Fig. 12 zeigt ein Ausführungsbeispiel für einen dreiarmigen Gelenkträger entsprechend Fig. 4. In diesem Prinzipschaltbild sind demgemäß drei Synchron-Sinus-Cosinus-Wandler 157, 158 und 159 vorgesehen. Ent-

0006499

- 16 -    VPA 78 P 5057 EUR

.sprechend dem Ausführungsbeispiel der Fig. 8 für die Resolverkette erfolgt Zuführung zu einem Multiplexer 160 mit nachgeschaltetem Analog-Digital-Wandler 161 .durch Direktverbindung mit dem Wandler 159 einerseits und Verbindung mit den Wandlern 157 und 158 über Summenbildner 162 und 163 andererseits.

Patentansprüche

1. Gerät zur Objektabtastung, insbesondere Ultraschall-Abtastgerät, mit Gelenkträger für wenigstens einen Abtastkopf, insbesondere Ultraschallkopf, und einer Einrichtung mit Winkelgebern in den Gelenken des Gelenkträgers zur Erfassung von Punkt- und/oder Richtungskoordinaten der Abtastung bei beliebiger Raumstellung des Abtastkopfes in Abhängigkeit von Winkelsignalen der Winkelgeber, g e k e n n z e i c h n e t   d u r c h wenigstens je einen Synchronmotor (17, 18 bzw. 52, 53, 54) vorzugsweise in jedem winkelzuerfassenden Gelenk (7, 9 bzw. 14, 15, 16) des Gelenkträgers (1a bzw. 1b) als Winkelgeber für den Sinus und/oder Cosinus des Winkelwertes des Drehwinkels ( $\alpha$ , $\beta$   bzw. $\alpha$ , $\beta$ , $\gamma$ ) der Motorachse sowie durch eine rein elektrische Zusammenschaltung einzelner oder mehrerer Synchronmotoren im Sinne der Bildung einer Drehmelder- oder Resolverkette oder einer gemischten Kette aus Drehmeldern und Resolvern zur Umwandlung von Sinus- bzw. Cosinuswerten erfaßter Drehwinkel in Summen und/oder Differenzen aus Einzelwinkeln und/oder in Sinus- bzw. Cosinusfunktionen solcher Winkelsummen bzw. Winkeldifferenzen.

2. Gerät nach Anspruch 1, d a d u r c h   g e - k e n n z e i c h n e t ,   daß den Signalausgängen der Kette aus Drehmeldern und/oder Resolvern Demodulatoren (27, 28, 32, 33 bzw. 114 bis 119) nachgeschaltet sind zur Demodulation der Ausgangssignale im Sinne der Erfassung der jeweiligen Winkelinformation.

3. Gerät nach Anspruch 2, d a d u r c h   g e - k e n n z e i c h n e t ,   daß die Demodulatoren eine Sample-and-Hold-Schaltung (45 bis 48 bzw. 114 bis 119) umfassen, die zur Demodulation der Ausgangssignale der Drehmelder- oder Resolverkette in Abhängigkeit vom Ein-

- 2 -    VPA 78 P 5057 EUR

gangswechselsignal (Vi) der Kette getastet ist.

4. Gerät nach Anspruch 3, d a d u r c h  g e - k e n n z e i c h n e t ,  daß die Ansteuerung der Sample-and-Hold-Schaltung in Abhängigkeit vom Eingangswechselsignal (Vi) bei Verwendung eines einfachen Sinusgenerators (49) als Wechselspannungsgeber über eine PLL-Schaltung (50) mit nachfolgender monostabiler Kippstufe (51) oder bei Verwendung eines Sinus/Cosinus-Generators (65) über einen Komparator (138) mit nachgeschalteter monostabiler Kippstufe (139) erfolgt.

5. Gerät nach einem der Ansprüche 1 bis 4, d a d u r c h  g e k e n n z e i c h n e t ,  daß zur Erfassung der Winkelinformationen an den Signalausgängen der Drehmelder- oder Resolverkette wahlweise Winkel-Wandler (SWW1 bis SWWn bzw. SWW12 bis SWWn2) oder Sinus-Cosinus-Wandler (SSC1 bis SSCn oder SSC12 bis SSCn2 bzw. 151, 152 bzw. 157, 158, 159) oder beide gleichzeitig anschaltbar sind.

6. Gerät nach einem der Ansprüche 1 bis 5, d a - d u r c h  g e k e n n z e i c h n e t ,  daß den Ausgängen der Drehmelder- oder Resolverkette Signalverarbeitungsglieder (34, 35 bzw. 149, 150 bzw. 155, 156bzw. 160 bis 163) zur Verarbeitung der Winkelsignale im Sinne der Bildung von Signalsummen- oder Signaldifferenzketten nachgeschaltet sind.

7. Gerät nach Anspruch 6, d a d u r c h  g e k e n n - z e i c h n e t ,  daß die Signalverarbeitungsglieder Integratoren (34, 35 bzw. 155, 156) zur Aufintegrierung der anfallenden Winkelinformationen im Sinne der Ermittlung der Punkt- und/oder Richtungskoordinaten der Abtastung umfassen.

8. Gerät nach Anspruch 7, d a d u r c h   g e -
k e n n z e i c h n e t ,   daß zur Vorgabe von Anfangsbedingungen den Integratoren (34, 35 bzw. 155,
156) Potentiometer oder Addierer (29, 30 bzw. 153, 154)
vorgeschaltet sind, deren Eingangsspannungen Ausgangssignale der Drehmelder- bzw. Resolverketten sind.

9. Gerät nach einem der Ansprüche 6 bis 8, d a -
d u r c h   g e k e n n z e i c h n e t ,   daß zur
Bildung von Teilsummen oder Teildifferenzen der Signalsummen- oder Signaldifferenzketten die Signalverarbeitungsglieder Summen- und/oder Differenzbildner (146,
147 bzw. 162, 163) umfassen.

10. Gerät nach einem der Ansprüche 6 bis 9, g e -
k e n n z e i c h n e t   d u r c h   eine Multiplexerschaltung (149 bzw. 160) zur Bildung von Signalsummen-
oder Signaldifferenzketten.

11. Gerät nach Anspruch 10, d a d u r c h   g e -
k e n n z e i c h n e t ,   daß den Multiplexerschaltungen Analog-Digital-Wandler (150 bzw. 161) nachgeschaltet sind.

FIG 1

FIG 2

78 P 5057    1/7

0006499

FIG 3

FIG 4

FIG 5

00006499

FIG 6

FIG 7

FIG 8

FIG 9

FIG 10

FIG 11

FIG 12

# EUROPÄISCHER RECHERCHENBERICHT

Europäisches Patentamt

| | EINSCHLÄGIGE DOKUMENTE | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | A 61 B 10/00 G 01 S 7/00 G 08 C 21/00 |
| | AT-B- 2 83 584 (INSTIUT) \* Seite 2, Zeilen 14-24; Figur 1 \* -- | 1 | |
| | US-A- 3 448 606 (MAGNAFLUX) \*Spalte 9, Zeilen 3-44; Figur 8\* -- | 1 | |
| | US-A- 3 439 530 (MAGNAFLUX) \*Spalte 4, Zeilen 44-62; Figur 3\* -- | 1 | |
| | US-A- 3 308 652 (SMITH) \*Spalte 5, Zeilen 21-26; Figur 2\* -- | 1 | RECHERCHIERTE SACHGEBIETE (Int. Cl.²) A 61 B 10/00. G 01 S 7/00, G 01 S 7/48, G 01 S 7/50 G 01 S 7/52 G 01 B 7/30 G 08 C 21/00 |
| D | DE-A- 1 573 745 (PICKER) -- | 1 | |
| A | DE-A1- 2 620 793 (AKADEMIET) -- | 1 | |
| A | DE-A1-2 724 998 (CGR) -- | 1 | |
| A | US-A- 3 944 798 (EATON) -- | 1 | |
| A | W.BOPP et al "Radar, Grundlagen und Anwendungen", 1962, FACHVERLAG SCHIELE & SCHÖN GMBH, Berlin \*Seiten 159-172\* ----- | | KATEGORIE DER GENANNTEN DOKUMENTE X: von besonderer Bedeutung A: technologischer Hintergrund O: nichtschriftliche Offenbarung P: Zwischenliteratur T: der Erfindung zugrunde liegende Theorien oder Grundsätze E: kollidierende Anmeldung D: in der Anmeldung angeführtes Dokument L: aus andern Gründen angeführtes Dokument &: Mitglied der gleichen Patent- familie, übereinstimmendes Dokument |

X Der vorliegende Recherchenbericht wurde für alle Patentansprüche erstellt.

| Recherchenort WIEN | Abschlußdatum der Recherche 22-08-1979 | Prüfer BURGHARDT |
|---|---|---|

EPA form 1503.1 06.78